Europäisches Patentamt

**European Patent Office**

**Office européen des brevets**

(11) Publication number: **0 266 968**
**A2**

(12) **EUROPEAN PATENT APPLICATION**

(21) Application number: **87309550.9**

(22) Date of filing: **29.10.87**

(51) Int. Cl.4: **A61K 9/06** , A61F 5/43 ,
A61L 15/03

(30) Priority: **03.11.86 US 926430**

(43) Date of publication of application:
**11.05.88 Bulletin 88/19**

(84) Designated Contracting States:
**AT BE CH DE ES FR GB GR IT LI LU NL SE**

(71) Applicant: **Cohen, Gérard G.**
**75 Rue Brillat-Savarin**
**F-75013 Paris(FR)**

Applicant: **Gottesmann-Cohen, J. Claire**
**75 Rue Brillat-Savarin**
**F-75013 Paris(FR)**

(72) Inventor: **Cohen, Gérard G.**
**75 Rue Brillat-Savarin**
**F-75013 Paris(FR)**
Inventor: **Gottesmann-Cohen, J. Claire**
**75 Rue Brillat-Savarin**
**F-75013 Paris(FR)**

(74) Representative: **Atkinson, Peter Birch et al**
**Marks & Clerk Suite 301 Sunlight House**
**Quay Street**
**Manchester M3 3JY(GB)**

(54) **Gelled ointment of vasodilating agent.**

(57) To a gelled ointment of a carboxyvinyl polymer, a water-soluble organic amine, a lower molecular weight alcohol, and water is added one or more active ingredients from the group consisting of procaine, theo phylline, nicotinic acid, vincamine, isoptine and, preferably, papaverine. The ointment is administered percutaneously to the human penis as a strong, smooth muscle relaxant of the penile arteries for diagnosis and/or therapy of erectile failure. The ointment is also deliverable upon the interior surface of a condom or by any transdermal system adaptable to the penis.

EP 0 266 968 A2

## GELLED OINTMENT OF VASODILATING AGENT

The present invention relates to a diagnostic and therapeutic method for human erectile failure.

Intracavernous injections of strong vasodilating agents, particularly papaverine, can result in strong vasodilation in the penile arteries (even when pathologic) and a diminished outflow from the cavernous bodies. This is discussed by Virag et al. in the article "Intracavernous Injection of Papaverine as a Diagnostic and Therapeutic Method in Erectile Failure", Angiology-Journal of Vascular Diseases, February, 1984, pp. 79-87. The next-following ten paragraphs are extracted from that paper.

The mechanism of erection and erectile failure is understood. During sexual stimulation, vasoactive intestinal polypeptide (VIP), a neurotransmitter which induces relaxation of the smooth muscles of the cavernous tissue, is released, whereupon the helicine arteries dilate to fill the widedly opened cavernous spaces. In this situation, the arterial pressure makes the intracavernous pressure (ICP) rise until a critical level of about 75 mmHg. This level seems to be necessary to obtain full rigidity by complete closure of the veinous outflow.

Erectile failure occurs if these haemodynamic mechanisms are not functioning. The reasons could be psychogenic, when the neural reflexes leading to the vascular reactions are absent or inhibited, or organic when the neurovascular penile structure is impaired. More often, erectile failure is caused by a mixture of organic and psychogenic components interacting to maintain impotence.

In a recent survey (cited by Virag et al.) of 220 cases of impotent males undergoing an extensive multidisciplinary evaluation, one or several organic causes were recognized in 80% of the patients. Among these, the arterial causes were prevalent (53%). Half of the patients demonstrated fragile psychologic structures. In a larger series (n = 440), the prevalence of the four main arterial risk factors (diabetes, hypertension, hypercholesterolemia, and smoking) were clear in cases of organic impotence. This suggests that the increasing rate of impotence, with age, should be attributed to diminished arterial inflow in the penile arteries.

A common situation is the patient's prolonged period of time to achieve a weaker erection than in his youth. These delayed and weaker erections' initiate a feat to fail in performing normal sexual intercourse. The performance anxiety (PA) acts as a stress-factor and enhances the organically based failure.

Psychological or behavioral therapies usually fail in such circumstances because they ignore the organic lesions. However, surgical procedures using revascularization or penile prosthesis are generally limited to patients having severe organic etiology. Many patients likely could avoid such procedures if a proper non-surgical therapy was available. Until the aforementioned study reported by Varig, it is believed that no clinical or experimental study had demonstrated the ability of any compound, given orally or intraveinously, to produce an erection.

Artificial Erection (AE) by intracavernous injection of heparinized saline is known to be used as a diagnostic approach during the evaluation of impotent patients. Several patients have reported improvement of their erections after such procedures. It is documented by arteriography and by Doppler studies that during AE vasodilation of the penile arteries occurs. Meanwhile, the ability to produce an erection, caused by the vasoactive drug papaverine given directly into the cavernous bodies, appears to have been discovered accidentally, then studied and reported. An evaluation of the impotent patients who improved by AE allowed the exclusion of placebo effect: no non-organic patients improved. They all apparently had various degress of arterial or mixed arterial and neurologic lesions. In such patients the onset of erections is impaired. The haemodynamic studies have demonstrated their ability to keep the blood inside the cavernous bodies, provided that a sufficient intracavernous pressure can be achieved.

In view of the above mentioned findings, Virag et al. reported a documented investigation into the effect on impotence by repeated intracavernous injection of papaverine associated with intracavernous infusion of saline, carried out in a series of patients with neurovascular impairment of their erectile function.

The investigation was performed on 45 men: 6 normally potent volunteers, 10 psychogenic, and 29 organic impotent males. The first two groups obtained erections within 10 minutes, lasting from 1 to 4 hours, after injection of 80 mg of papaverine. Those belonging to the organic group experienced delayed, weaker and shorter erections, related to various degrees of arterial and/or veinous and neurologic lesions.

Haemodynamic and radiologic studies were done concomitantly and showed a strong vasodilation in the penile arteries (even when pathologic) and diminished outflow from the cavernous bodies. Subsequently, a pilot study was performed in a series of 63 patients, suffering from various degrees of angiogenic impotence, in order to study the therapeutic effect of repeated intracavernous injections of papaverine completed by intracavernous infusion of heparinized saline. Erections improved significantly in 66% of the patients with a mean follow up of one year.

Finally, Virag et al. points out that the ability to produce a full erection by intracavernous injection of papaverine presents an ethical problem concerning its use and abuse as a home treatment for psychogenic and neurovascuar impotence, even for normally potent patients who might desire a prolonged and more frequent erection.

Possibly due to promising results obtained in diagnosing and treating erectile failure by injection of vasodilating agents, additional papers relating to this method are now appearing in medical journals. The following English language articles appear in the 1986 Journal of Urology. In March, T.F. Lue, H. Hricak, R.A. Schmidt, and E.A. Tanasho published "Functional evaluation of penile veins by cavernososraphy in papaverine-induced erection." In April, J.S. Cameron, L.M. Duffy, and P.H. Lange published "Intracavernous drug-induced erections in the management of male erectile dysfunction: experience with 100 patients." In May, J.C. Abber, T.F. Lue, B.R. Orvis, R.D McClure and R.D. Williams published "Diagnostic tests for impotence: a comparison of papaverine injection with the penile-brachial index and nocturnal penile tumescense monitoring." In July, K.P. Juenemann, T.F. Lue, G.R. Fournier, Jr., and E.A. Tanasho published "Hemodynamics of papaverine-and phentolamine-induced penile erection."

Demonstrating international interest in the subject, a January 1985 article was published in Japanese in Hinyokika Kiyo by T. Takamura, H. Hashimoto, M. Miyata, Y. Nakata, H. Osanai, and S. Yaehiku, entitled "Treatment of organic impotence, Self-administered intracavernous injection of papaverin HCl (a preliminary report)" in English translation. None of the papers are concerned with a gelled ointment of vasodilating agent, or with the diagnostic and therapeutic method involving percutaneous administration of a vasodilating agent, which will be seen to be subjects of the present invention.

Also relevant to the present invention are prior art compositions of gelled ointments for topical percutaneous application. One such ointment is an "Antiinflammatory Analgesic Gelled Ointment" described in U.S. Patent No. 4,533,546 to Kishi et al. The ointment comprises an active ingredient (particularly a phenylacetic acid type antiinflammatory analgesic agent), a carboxyvinyl polymer in an amount sufficient for gel formation, a water-soluble organic amine, a lower alcohol and water in order to produce a compound having a pH in the range of from 7.0 to 9.0.

Finally, the present invention will be seen to extend to a condom-based system for delivery of a medicine topically to the penis. It is known that condoms may be employed in combination with certain gels and creams. These agents are normally pre-applied to the exterior surface. Commonly used gels and creams include spermicidal, anesthetic, analgesic, and prophylactic active agents. Although prior art agents serve some purposes, these purposes are not believed to have included diagnosis and therapy of haemodynamic function within the male human.

It may further be noted that it is known to apply certain active agents, such as scopolamine for the prevention of motion sickness (sea sickness) or drugs for the control of hypertension, by patches which affix to the skin. The administration of medicines by this technique is percutaneous and transcutaneous, often called "transdermal". A medicine previously administered transdermally by patches which involves haemodynamic activity of the heart is nitroglycerine, which is used in cardiology.

The present invention has aspects at several levels in the administration of medicines which are particularly directed to diagnosis and pharmacotherapy of erectile failure. In the past, administration of vasodilating agents, including papaverine, has transpired by intracavernous injection. At a basic level the present invention concerns the preparation of a medical compound --mainly, a gelled ointment of a vasodilating agent --and a method for using the preparation --mainly, percutaneous administration of the compound to the penis. At a higher level the present invention concerns a safe and reliable delivery system exercisable by non-medically-trained personnel for self-delivery of potent vasodilating agents --mainly, a condom or a transdermal patch to which a gelled ointment of a vasodilating agent is applied.

In accordance with the present invention an ointment, nominally gelled, contains a vasodilating agent, or smooth muscle relaxant, as an active ingredient. The gelled ointment contains the vasodilating agent, nominally from .5 to 5% by weight, plus a carboxyvinyl polymer in the range of .5 to 3% by weight, a water-soluble organic amine in the range of .5 to 5% by weight, a lower molecular weight alcohol in the range of 20 to 50% by weight, and water in the range of 40 to 80% by weight. The vasodilating agent may particularly be selected from the group consisting of papaverine, procaine, theo phylline, nicotinic acid, isoptine, and vincamine. The vasodilating agent is preferably papaverine preferably incorporated in the gelled ointment at .5 to 2.5% by weight.

Further in accordance with the present invention, administration of a vasodilating agent is transcutaneous or percutaneous. The gelled ointment of a vasodilating agent is topically percutaneously applied to the penis. By the percutaneous administration of the ointment a vasodilation of the penile arteries and a diminished outflow from the cavernous bodies are obtained. Similarly to results obtained with normally potent, psychogenic, and organic impotent males upon the intracavernous injection of papaverine, percutaneous administration of the gelled ointment of a vasodilating agent induces erections.

Further in accordance with the present invention, are presented delivery systems for the percutaneous application of a vasodilating agent. The delivery systems are, for the purposes of diagnosis and therapy of erectile failure, exercisable by a non-medically-trained consumer user. Any system delivering a vasodilating agent to a consumer places a powerful drug in hands where it may be used, misused, or abused. It is therefor useful that the potency, dosage, and manner of use of the vasodilating agent should, to such extent as is possible, be very tightly controlled. In accordance with the present invention, one preferred delivery system for the use of vasodilating agents in diagnosis and therapy of erectile failure consists of a condom containing the gelled ointment of a vasodilating agent on its interior surface. An alternative preferred delivery system is a patch which is affixed to the skin and which slowly transdermally infuses a vasodilating agent. The potency and amount of each administration of the vasodilating agent within each delivery system is tightly controlled. Abuse is diminished because dosage strengths and amounts are not within the control of the consumer user. Integration of the ointment of vasodilating agent with the condom discourages misuse which occurs when the ointment is applied to other than the target penis body member. Likewise to discouraging misuse, the patch may be specially contoured to induce placement upon the penis or other suitable location of the male body.

It is known that intracavernous injection of a vasodilating agent, particularly papaverine, can serve as a medical diagnostic and therapeutic method in erectile failure. The potency, amount, frequency, and manner of injecting by syringe powerful vasodilating agents must be strictly controlled by medically trained personnel. However, the results obtained in affecting the onset of erections within approximately 10 minutes followed by from 1 to 4 hours of penile rigidity are of potential widespread benefit to some males suffering impotence. A safe and effective system for delivering the benefit of vasodilating agents to the consumer user is desired.

The present invention concerns a medicine and its preparation, a manner of use of the medicine, and a delivery system for delivering the medicine to the consumer user so that he will use it in the prescribed manner. The medicine is a gelled ointment of a vasodilating agent. The manner or use of the gelled ointment is by percutaneous application, particularly topically to the penis. The delivery system for the medicine is either a condom or a transdermal patch which contain upon their surfaces in contact with the skin a vasodilating agent, nominally in the form of the gelled ointment.

In accordance with that aspect of the present invention concerning a medicine and its preparation, an ointment containing one or more active vasodilating agents, or smooth muscle relaxants, is prepared. For percutaneous administration, and for bioavailability by the route of percutaneous administration, the active agent has to be soluble both in water and organic solvent. It is best to have a partition coefficient near 1.0. Active ingredients of the group "smooth muscle relaxant (as such group is defined in the general monography of the U.S.P.) are preferred.

The active agent(s) is(are) preferably drawn from among the vasodilating agents (or mixtures thereof) listed in Table I.

TABLE I   Papaverine
    Procaine
    Theo Phylline
    Nicotinic Acid
    Isoptine
    Vincamine

The active agent is preferably papaverine hydrochloride ("HCl") in the range of .5 to 5% by weight. The papaverine HCl is optimally in the range of .5 to 2.5% by weight and more optimally at 1% by weight.

Remaining, non-active, ingredients of the medical ointment are preferably chosen so as to make the resultant ointment a homogenous, clear, and stable gel with good compatibility to genital tissue. The gelled ointment is preferably formulated of a carboxyvinyl polymer, a water-soluble organic amine, a lower molecular weight alcohol, and water. The carboxyvinyl polymer is within the carbomer general monography of the U.S.P. It is preferred to precisely use Carbopol 934. Any compound or polymer which is useable in the manufacture of a gel which is compatible with human tissue may alternatively be used.

This polymer is acid when dispersed with vigorous agitation in water. After dispersion and agitation for approximately 6 hours, it is neutralized with a basic substance to form a gel. The amount of the carboxyvinyl polymer is preferably within the range of 0.5 to 3.0% by weight. It is more preferred to use 1% by weight.

A water-soluble organic amine serves as the neutralizing agent within the gel. It is preferred that NaOH or KOH should not be used because of the further presence of alcohol. It is preferred to use organic amines and preferred lower alkanol amines. Suitable organic amines are listed within the following Table 2. This table, however, is not to be taken as limiting.

TABLE 2 Monoamines =
Monoethanolamine
Monopropanolamine
Monoisopropanolamine
Diamines =
Diethanolamine
Dipropanolamine
Disopropanolamine
Dibutanolamine
Tramines =
Tri Methanolamine
Tri Ethanolamine
Tri Propanolamine

It is preferred to precisely use Tri Ethanolamine. The quantity used is that which is required to neutralize the carbomer. It must preferably be used in an amount which produces a pH normally in a range of 6 to 8 or more. The pH range is preferably 6.5 to 7.2 for obtaining the most homogenous, clear and stable gel with good skin tolerance and compatibility.

Depending on the type of amine, the amount of active ingredient and which individual active ingredient is used, and the quantity of carbopol, the amount of the amine is between 0.5 and 5.0% by weight. It is preferred to be between 0.5 to 2.5% by weight. The amine is more preferably 2.5% by weight.

Alcohol is used as a carrier, although other compatible liquid and semiliquid pharmaceutical carriers are within the scope of this invention. It is preferred to use a lower molecular weight alcohol such as methanol, ethanol, propanol, isopropanol, butanol, and/or amyl alcohol. Because of skin tolerance and compatibility, ethanol 95% is especially preferred. If ethanol is not used, isopropanol is also especially preferred. The amount of alcohol used depends on the active ingredient and its solubility. The preferred range is between 20.0 and 50.0% by weight to assure the best percutaneous absorption. The more preferred concentration is 30.0% by weight of ethanol 95%.

Water is also used as a carrier. The amount of water depends on the active agent and the quantity of alcohol used. It may vary between 50.0 to 80.0% by weight because a large quantity of water is necessary to disperse carbopol and make the gel stable and convenient to apply. A concentration of 65.5% is highly preferred.

The summary ingredients of the gelled ointment of a vasodilating agent of the present invention are as listed in the following Table 3.

## TABLE 3

### Proportion of Ingredients Used

| Ingredient | General Range | Optimum Range |
|---|---|---|
| | % by Weight | |
| Active Agent | | 0.5 to 5 |
| Carbyoxyvinyl Polymer | 0.5 to 3 | 0.5 to 2 |
| Organic Amine | 0.5 to 5 | 0.5 to 2.5 |
| Lower Alcohol | 20.0 to 50.0 | 25.0 to 35.0 |
| Water | | 40.0 to 80.0 |

One highly preferred formulation is as list the following Table 4.

## TABLE 4

### Precise Formulation % By Weight

| | |
|---|---|
| Papaverine Hydrochloride | 1 |
| Carbopol 934 | 1 |
| Triethanolamine | 2.5 |
| Ethanol 95% | 30 |
| Water | 65.5 |
| | 100.0 |

For this preferred precise formulation the pH = 7 and the alcohol degree = 40.

The preferred manner of preparation of the gelled ointment of a vasodilating agent of the present invention is in accordance with the steps of the following Table 5.

## TABLE 5

### Preparation

1. 1 gram of carbopol is dispersed in 50 ml. water with vigorous agitation - leave approximately 4 to 6 hours for complete hydration.
2. Dissolve active ingredient in a separate vessel with remainder of water and alcohol by stirring.
3. Add the complete solution of active ingredient in a tank containing the carbopol dispersion by sweeping agitation.
4. Mix and homogenize for 10 to 15 minutes.
5. Neutralize with triethanolamine by sweeping agitation.

In accordance with the manner of use aspect of the present invention, the gelled ointment of a vasolidating agent is applied to the skin of the penis. The administration and absorption of the active ingredient vasodilating agent is percutaneous, or transcutaneous. In order to assess the efficacy and safety of this percutaneous absorption, in vitro diffusion tests were conducted in a FRANZ GLASS diffusion cell with hairless rats' skin. The tests were conducted with 10 ml of the different preparations at 1% concentration. The mean results of six determinations in such tests performed with application of 100 micrograms of papaverine hydrochloride on the skin are expressed in the following Table 6.

The results in Table 6 are expressed in micrograms per cm$^2$ during each period of time and are cumulative.

## TABLE 6

### In Vitro Diffusion Test Results

|  | Period After Application | | | |
|---|---|---|---|---|
|  | 2 hours | 4 hours | 8 hours | 24 hours |
| Gel Formulation | 0.083 | 0.254 | 0.423 | 1.038 |
|  |  | 0.337 | 0.760 | 1.798 |
| Solution (water & alcohol | 0.061 | 0.148 | 0.321 | 0.934 |
|  |  | 0.209 | 0.530 | 1.464 |
| Ointment (lanolin + petroleum) | 0.012 | 0.042 | 0.092 | 0.083 |
|  |  | 0.054 | 0.146 | 0.229 |

Skin irritation tests were additionally performed with papaverine. Results of a skin irritation test on male rabbit skin after application of 500 mg. of gel with 1% active ingredient for 24 hours with occlusive protection, and after 48 and 72 hours, are shown in the following Table 7:

## TABLE 7

### Skin Irritation Test Results

|  | Period After Initial Application | | | | | |
|---|---|---|---|---|---|---|
|  | 24 hours | | 48 hours | | 72 hours | |
| Untreated | | | | | | |
| - erythema | - | - | - | - | - | - |
| - oedema | - | - | - | - | - | - |
| - desquamation | - | - | - | - | - | - |
| Treated | | | | | | |
| - erythema | + | + | - | - | - | - |
| - oedema | - | - | - | - | - | - |
| - desquamation | - | - | - | - | - | - |

The character "-" within Table 7 means no skin irritation was observable, and the character "+" means that minor skin changes were observable but were of an insignificant and non-irritating nature but showing the local vaso-activity of the preparation.

The haemodynamical effect of application of the gelled ointment of vasodilating agent to the penis must be understood in context of the mechanism of erection. When sexual stimulation occurs in the male, a neurotransmittal induces relaxation of the smooth muscles of the cavernous tissue and the helicine arteries dilate to fill the widely opened cavernous spaces. In this situation the level of intracavernous pressure has to be about 75 millimeters Hg in order to obtain full penile rigidity and complete closure of the venous outflow.

Erectile failure occurs in different situations. These include hormonal deficiency in testosterone plasmatic level, psychogenic problems, and organic problems with attendant dysfunction of haemodynamic mechanism. For the most part, male erectile failure has two components in different proportions, one hormonal or organic and one psychogenic, interacting one with the other to maintain impotence. When organic problems other than those due to a hormonal situation exist, then the arterial causes are predominant.

Prior therapeutic solutions to erectile failure include psychological therapy, surgery, and artificial erection by intracavernous injections of a heparnized saline solution with an active ingredient. Haemodynamics problems in erection have been demonstrated. The ability in the presence of haemodynamics problems to keep the blood inside the cavernous bodies has also been demonstrated, provided that sufficient intracavernous pressure can be achieved.

That prior solution to erectile failure which is seemingly most pertinent to the present invention was the intracavernous injection of a vasodilator. A dosage of from 40 to 160 mg. of the active ingredient papaverine was injected intracavernously. Resulting penile erections had a duration of approximately 2 hours. There was a latency to obtain maximum changes of approximately 10 to 15 minutes.

The present invention avoids painful injections and problems of time delay. A local, percutaneous application on the penis of a gel containing a vasodilating agent serves as a strong smooth muscle relaxant of the penile arteries. As might be anticipated from use of the same active ingredient as was previously used in injections of a vasodilating agent, penile erections obtained by use of the gelled ointment of vasodilating agent in accordance with the present invention are roughly equivalent in onset and duration to those previously induced by intracavernous injection. The percutaneous application in accordance with the present invention serves to diagnose erectile failure in that if erection is not achieved then organic impotence is suspect. The percutaneous application in accordance with the present invention serves as therapy to erectile failure by according penile erections when erectile failure was due to psychogenic and to some organic causes.

Such problems as are presented by the gelled ointment, and the manner of its use, in accordance with the present invention go more to safety than any insufficiency of results. It must always be remembered that a vasodilating agent, even when contained within a gelled ointment, is a powerful drug. Consequently, and in accordance with the delivery system aspect of the present invention, it should be understood that it is useful to establish a system for safely and ethically delivering the considerable benefits of a vasodilating agent to the therapy of erectile failure. The male users of the gelled ointment of the present invention will predominantly have no medical training whatsoever. There will exist all normal propensities for product overuse, misuse, and abuse. Consequently, it may be envisioned that bulk supplies of gelled ointment of vasodilating agent will probably be obtainable, at least in the U.S., only by physician's prescription.

Even as regards a consumer user who is attempting to be most cautious and circumspect in his use of the gelled ointment of vasodilating agent, it is very helpful to provide a unitary dosage of controlled potency and amount which is targeted on the preferred application area. This control and selectivity is obtained, in accordance with the preferred delivery system aspect of the present invention, by emplacing the gelled ointment of a vasodilating agent upon the interior surface of a condom or upon the surface of a transdermal patch. An individually packaged condom unit, incorporating a predetermined strength and amount of active vascodilator ingredient, is intended to be used as a sheath upon the penis. Since the purpose of applying the gelled ointment of a vasodilating agent to the skin of the penis is separate, and separable, from the purposes of preventing venereal infection or conception during coitus, then the condom may be modified in form, as by being substantially open over the glans penis. Therefor it will be recognized that the use of the word "condom" within this application means "condom-like device". The patch likewise contains a controlled dosage of vasodilating agent, which may again be in the form of the gelled ointment of vasodilating agent. The patch is intended to be placed on the penis, or at least near to the penile arterial blood flow.

It is also important to recognize that many improvements upon the combination of a gelled ointment of vasodilating agent and a condom are possible. It may be useful that the vasodilating agent should be impregnated within a one surface of the condom or the patch so that it may not easily be scraped off for use or misuse in other bodily areas. It may be important that the condom impede, or disable, being reversed. It may be useful that the friction of the internal surface of the condom be increased, including by alteration of the composition of the gelled ointment. It may be useful to construct a double walled condom with timed release of a contained vasodilating agent, possibly in gelled ointment form, onto the skin of the penis. The use of a condom or of a transdermal patch in combination with a gelled ointment of vasodilating agent are not merely combinations of convenience, but are intended to address very real issues regarding the use of a potent medication producing significant effects.

One alternative preferred delivery system in accordance with the present invention is to implace a controlled dosage of a vasodilating agent within a patch which is affixed to the skin. The gelled ointment of vasodilating agent may be employed in a combination with an adhesive patch, or adhesive bandage, in a like manner to the dressing of a wound while employing an antibiotic ointment. Alternatively, the vasodilating agent may be perfused within a "transdermal" patch, from which it will migrate transdermally through the skin, in accordance with known techniques for the transdermal administration of medication. The patch may even be sized and contoured to encourage placement on the staff of the penis or other location, and to discourage misuse such as ingestion.

Importantly to the delivery system concept of the present invention, it will be recognized that at least two systems for delivery of a potent vasodilating agent in controlled dosage and amount are possible. Both the condom and the adhesive or transdermal patch may generically be described as a device, or matrix, by which a vasodilating agent may be controllably administered to the body by untrained personnel.

In accordance with the preceding discussion, the present invention should be broadly perceived to have aspects of a medical compound, of a method of administering such medical compound, and of a delivery system for use of the medical compound in accordance with the administration method. It should particularly be recognized that the present invention is not merely a gelled ointment of vasodilating agent. The gelled ointment is merely a preferred component compound of the invention which is used within the preferred method of the invention which preferred method is induced by the preferred delivery system of the invention. The invention might have instead been called "A System for Controlled Delivery of Vasodilating Agents". The invention might have instead been called "A Method of Delivering Vasodilating Agents to the Body".

Correspondingly, the present invention should be interpreted only by the scope of the following claims, and not merely in context of those particular preferred embodiment implementations and methods within which the present invention has been taught.

## Claims

1. A method of administering a smooth muscle relaxant to the human penile arteries comprising:
mixing a smooth muscle relaxant in a compound as the active ingredient thereof; and
applying the compound percutaneously to the human penis.

2. The method according to claim 1 wherein the compound is an ointment.

3. The method according to claim 1 wherein the smooth muscle relaxant reagent is a member of the group consisting of papaverine, procaine, theo phylline, nicotinic acid, isoptine and vincamine.

4. The method according to claim 2 wherein the ointment is gelled.

5. The method according to claim 1 further comprising:
holding the compound in percutaneous contact with the penis by overlaying the compound with a condom sheathing the penis.

6. The method to claim 1 further comprising:
holding the compound in percutaneous contact with the penis by overlaying the compound with an adhesive patch.

7. The method according to claim 1 wherein the mixing is of the smooth muscle relaxant reagent papaverine in an amount from .5 to 5% of the compound by weight.

8. A method of delivering a vasodilating agent into the hands of a non-medically-trained consumer user directed to simultaneously inducing 1) safe use by the consumer user and 2) use topically directed to the penile arteries, the method comprising:
mixing a vasodilating agent in an compound as the biologically active ingredient thereof;
applying the compound to the surface of a matrix; and
delivering the matrix into the hands of a non-medically-trained consumer user with instructions that the user is directed to place that surface of the matrix to which the compound is applied in contact with the skin of the body proximate the penile arteries.

9. The method according to claim 8 wherein the applying is to the inside surface of a condom matrix, and wherein the delivering is of the condom matrix with instructions to fit the condom to the skin of the penis.

10. The method according to claim 8 wherein the applying is to an adhesive patch matrix, and wherein the delivery is of the adhesive patch with instructions to affix the patch to the skin at or near the penis.

11. The method according to claim 8 wherein the mixing is of an ointment compound, and wherein the applying is of an ointment compound.

12. The method according to claim 7 wherein the vasodilating agent is a member of the group consisting of papaverine, procaine, theo phylline, nicotanic acid, isoptine and vincamine.

13. The method according to claim 7 wherein the ointment is gelled.

14. The method according to claim 7 wherein the mixing is of the vasodilating agent papaverine in an amount from .5 to 5% of the ointment by weight.

15. A method of the administration of a smooth muscle relaxant to the human penile arteries comprising:

applying a compound containing an active ingredient of a smooth muscle relaxant to the interior surface of a condom, and

emplacing the condom about the human penis so that the active ingredient is percutaneously absorbed.

16. The method according to claim 15 wherein the compound is an ointment.

17. The method according to claim 15 wherein the compound is a gelled ointment.

18. A delivery system apparatus for delivering a vasodilating agent as medicine to the penile arteries, the medicine delivery system apparatus comprising:

a condom; and

an ointment containing an active ingredient of a vasodilating agent applied to the interior surface of the condom.

19. A delivery system apparatus for delivering a vasodilating agent as medicine to the penile arteries, the medicine delivery system apparatus comprising:

a transdermal patch; and

a vasodilating agent suffusing the patch and controllably released therefrom.

20. The medicine delivery system apparatus of claim 18 wherein the ointment is consisting essentially of:

a vasodilating agent in the range of .5 to 5% by weight;

a carboxyvinyl polymer in the range of .5 to 3% by weight;

a water-soluble organic amine in the range of .5 to 5% by weight;

a lower molecular weight alcohol in the range of 20 to 50% by weight; and

water in the range of 40 to 80% by weight.

21. The medicine delivery system according to claim 18 wherein the vasodilating agent is consisting of one or more members of a group consisting of papaverine, procaine, theo phylline, nicotinic acid, isoptine, and vincamine.

22. The medicine delivery system according to claim 19 wherein the vasodilating agent is consisting of one or more members of a group consisting of papaverine, procaine, theo phylline, nicotinic acid, isoptine, and vincamine.

23. The medicine delivery system according to claim 18 wherein the vasodilating agent is papaverine.

24. The medicine delivery system according to claim 19 wherein the vasodilating agent is papaverine.

25. The medicine delivery system according to claim 20 wherein the vasodilating agent is papaverine, wherein the carboxyvinyl polymer is Carbopol 934, wherein the organic amine is tri ethanolamine, and wherein the alcohol is ethanol.

26. An apparatus for administration of muscle relaxants to the human penile arteries comprising:

a condom, and

a gelled ointment containing an active ingredient of a smooth muscle relaxant upon the interior surface of the condom.

27. The apparatus according to claim 26 wherein the smooth muscle relaxant is from the group consisting of papaverine, procaine, theo phylline, nicotinic acid, isoptine, and vincamine.

28. The apparatus according to claim 26 wherein the smooth muscle relaxant is .5 to 5% by weight of the gelled ointment.

29. The apparatus according to claim 28 wherein the smooth muscle relaxant is papaverine.

30. A medical compound for percutaneous administration consisting essentially of:

a vasodilating agent in the range of .5 to 5% by weight;

a carboxyvinyl polymer in the range of .5 to 3% by weight;

a water-soluble organic amine in the range of .5 to 5% by weight;

a lower molecular weight alcohol in the range of 20 to 50% by weight; and

water in the range of 40 to 80% by weight.

31. The medical compound according to claim 30 wherein the vasodilating agent is consisting essentially of or more members of a group consisting of papaverine, procaine, theo phylline, nicotinic acid, isoptine, and vincamine.

32. The medical compound according to claim 30 wherein the vasodilating agent is papaverine.

33. The medical compound according to claim 30 wherein the vasodilating agent is papaverine, wherein the carboxyvinyl polymer is Carbopol 934, wherein the organic amine is tri ethanolamine, and wherein the alcohol is ethanol.

34. An improvement to a medical compound for percutaneous administration, the improvement comprising:

addition of papaverine to the amount of .5% to 5% by weight of the compound.

35. To a gelled ointment having a carboxyvinyl polymer in an amount sufficient for gel formation,

a water-soluble organic amine,

a lower molecular weight alcohol, and

water,

the addition of an active ingredient, the added active ingredient to the gelled ointment consisting essentially of:

one or more ingredients of the group of smooth muscle relaxants consisting of papaverine, procaine, theo phylline, nicotanic acid, isoptine, and vincamine.

36. The added ingredient of claim 35 wherein the one or more ingredients are in the amount of .5 to 5% by weight in the gelled ointment.

37. A medical compound according to claim 30 consisting essentially of, by weight, 1.0% papaverine hydrochloride, 30.0% ethanol 95%, 1.0% carbopol 934, 2.5% ethanolanine, and 65.5% water.